# FASCICULE DE BREVET EUROPEEN

(11) **EP 1 133 354 B1**
(45) Date de publication et mention de la délivrance du brevet: **15.02.2006**
(21) Numéro de dépôt: 99953840.8
(22) Date de dépôt: 11.10.1999
(51) Int. Cl.: B01J 23/26, C07C 17/20, C07C 17/21, C07C 19/08

(54) **CATALYSEUR ET PROCEDE D'HYDROFLUORATION**
KATALYSATOREN UND VERFAHREN ZUR HYDROFLUORIERUNG
HYDROFLUORINATION CATALYST AND METHOD

(30) Priorité: 12.10.1998 BE 9800732
(43) Date de publication de la demande: 19.09.2001
(73) Titulaire: SOLVAY (Société Anonyme), 1050 Bruxelles (BE)
(72) Inventeur: WILMET, Vincent, B-1301 Wavre (BE); LEJEUNE, Georges, B-1080 Bruxelles (BE)
(74) Mandataire: Jacques, Philippe
(86) Numéro de dépôt international: PCT/EP1999/007782
(87) Numéro de publication internationale: WO 2000/021660

(56) Documents cités:
- EP-A- 0 554 165
- EP-A- 0 657 409
- EP-A- 0 776 878
- US-A- 5 475 167
- KIRK-OTHMER, ENCYCLOPEDIA OF CHEMICAL TECHNOLOGY * page 321 *

## Description

La présente invention concerne un catalyseur d'hydrofluoration à base d'oxyde de chrome, utilisé notamment pour transformer des hydrocarbures halogénés sous l'action du fluorure d'hydrogène.

De très nombreux catalyseurs ont été décrits pour la réaction d'hydrofluoration d'hydrocarbures halogénés aliphatiques sous l'action du fluorure d'hydrogène. On a cité le plus souvent des oxydes ou des halogénures de chrome, d'aluminium, de titane, de nickel, d'étain, d'antimoine ou d'autres métaux, utilisés comme tels ou déposés sur un support tel que du charbon actif, du graphite ou de l'alumine.

Parmi les catalyseurs au chrome largement utilisés, on trouve principalement des fluorures de chrome, des oxyfluorures de chrome et des oxydes de chrome, principalement le sesquioxyde de chrome (Cr₂O₃).

Le brevet canadien CA 861572 décrit la synthèse et l'utilisation d'oxyde de chrome anhydre comme catalyseur dans des réactions d'hydrofluoration d'hydrocarbures chlorés ou bromés. Aucune information n'est toutefois donnée quant à la pureté du catalyseur obtenu selon les procédés de préparation décrits dans le brevet.

La demande de brevet WO 92/19576 décrit l'utilisation d'oxyde de chrome comme catalyseur d'hydrofluoration en présence de fluorure d'hydrogène ainsi que sa préparation par décomposition thermique de dichromate d'ammonium. La présence de traces de métaux alcalins, et plus particulièrement de potassium, nuit fortement à l'activité de ce catalyseur.

Il apparaît que l'activité de l'oxyde de chrome comme catalyseur d'hydrofluoration est variable notamment en fonction de son procédé de préparation, de sa surface spécifique, de son état de cristallinité, de l'étage d'oxydation du chrome ou de son caractère amorphe sans toutefois qu'une explication cohérente quant à son activité ne soit fournie.

La demanderesse a trouvé que l'activité catalytique d'un catalyseur à base d'oxyde de chrome est fortement dépendante de la quantité de sels d'ammonium présente dans le catalyseur. Plus particulièrement, la demanderesse a trouvé que l'activité catalytique d'un tel catalyseur est inversement proportionnelle à la quantité de sels d'ammonium présents comme impureté dans le catalyseur.

Un objet de la présente invention consiste dès lors à fournir un catalyseur d'hydrofluoration à base d'oxyde de chrome contenant des sels d'ammonium, la teneur en ions ammonium étant inférieure ou égale à 0,2 % en poids.

Les sels d'ammonium présents dans le catalyseur peuvent notamment se présenter sous la forme d'un halogénure d'ammonium tel que le chlorure d'ammonium ou le fluorure d'ammonium, ou sous la forme d'un autre sel d'acide minéral ou organique tel que le nitrate d'ammonium, le chromate d'ammonium, le bichromate d'ammonium ou l'acétate d'ammonium. D'excellents résultats sont obtenus avec un catalyseur dont la teneur en ions ammonium est inférieure ou égale à 0,1 % en poids. Des résultats particulièrement intéressants sont obtenus avec un catalyseur dont la teneur en ions ammonium est inférieure ou égale à 0,05 % en poids.

Par convention, dans la présente description, les valeurs mentionnées pour la teneur en sels d'ammonium dans le catalyseur selon l'invention se rapportent à la teneur en ions NH₄⁺ par rapport à la teneur en chrome du catalyseur, exprimée sous la forme de Cr₂O₃.

L'oxyde de chrome utilisé dans le catalyseur selon la présente invention peut présenter une surface spécifique variable supérieure ou égale à 20 m²/g et inférieure ou égale à 500 m²/g, déterminée selon la méthode BET (Brunauer Emmet Teller). Généralement, le volume poreux du catalyseur, déterminé selon la méthode par adsorption d'azote, est supérieur ou égal à 0,05cm³/g et inférieur ou égal à 1 cm³/g. Le catalyseur peut être totalement amorphe ou totalement cristallisé, de même qu'il peut être partiellement amorphe et partiellement cristallisé. L'oxyde de chrome dans le catalyseur selon l'invention est généralement essentiellement à l'étage d'oxydation III mais le catalyseur peut également contenir des quantité variables de chrome se trouvant à un étage d'oxydation supérieur à III comme par exemple du chrome VI.

Typiquement, il peut être synthétisé selon un des procédés connus par l'homme du métier et plus particulièrement soit par réduction de l'oxyde de chrome (VI) (CrO₃) par un alcool tel que l'éthanol, soit par déshydratation à température élevée d'un gel d'hydroxyde de chrome (III), soit encore par pyrolyse du dichromate d'ammonium. Dans ce dernier cas, l'oxyde de chrome obtenu lors de l'étape de pyrolyse à haute température (généralement supérieure à 500 °C) est le plus souvent refroidi sous un courant d'air et lavé à plusieurs reprises jusqu'à ce qu'il n'y ait plus de trace d'ions ammonium dans la solution de lavage.

Dans le catalyseur selon la présente invention, l'oxyde de chrome peut être utilisé soit comme tel, sous forme massique, soit il peut être déposé sur un support tel que du charbon actif, du graphite, de l'alumine, de l'alumine fluorée, de l'oxyde de magnésium,... Le catalyseur selon l'invention est de préférence constitué d'oxyde de chrome sous forme massique.

Il peut en outre contenir d'autres métaux ou des sels d'autres métaux et leurs mélanges à titre de co-catalyseurs. Parmi les métaux ou les sels de métaux généralement utilisables, on peut citer, par exemple, le cobalt, le titane, le manganèse, l'étain, l'antimoine, le nickel ou le zinc ainsi que leurs sels et leurs oxydes. Les dérivés de métaux peuvent être incorporés au catalyseur au chrome selon divers procédés tels que l'imprégnation de l'oxyde de chrome par un composé de métal, par coprécipitation de précurseurs ou par mélange et broyage de composés de métaux solides.

Il est généralement avantageux de calciner le catalyseur avant usage. Classiquement, cette calcination se fait sous un courant de gaz inerte à une température supérieure ou égale à 200 °C et inférieure ou égale à 600 °C. Avantageusement, la température de calcination est supérieure ou égale à 250 °C et inférieure ou égale à 450 °C. Le gaz inerte est généralement choisi parmi l'azote ou les gaz rares tels que l'hélium, l'argon ou le néon. Pour des raisons économiques, l'azote est préféré. La durée de calcination est habituellement comprise entre 2 heures et 20 heures. Avantageusement, la durée de calcination est supérieure ou égale à 4 heures et inférieure ou égale à 16 heures. Le durée de calcination est de préférence supérieure ou égale à 6 heures et inférieure ou égale à 14 heures.

Généralement, le catalyseur est prétraité par du fluorure d'hydrogène avant d'être mis en oeuvre dans une réaction d'hydrofluoration. On pense que ce prétraitement convertit l'oxyde de chrome qui se trouve en surface en oxyfluorure de chrome. En général, ce prétraitement s'effectue dans un réacteur, habituellement celui qui sert aux réactions d'hydrofluoration selon l'invention, en faisant passer du fluorure d'hydrogène sur l'oxyde de chrome calciné et séché de façon à saturer l'oxyde de chrome par le fluorure d'hydrogène. Ce prétraitement se déroule habituellement sur une durée allant de 15 à 300 minutes à une température comprise généralement entre 200 et 700 °C. Ce prétraitement est souvent utile mais il n'est pas essentiel pour la bonne marche du procédé selon la présente invention.

Quel que soit le mode de préparation de l'oxyde de chrome, il est particulièrement avantageux que le prétraitement au fluorure d'hydrogène soit effectué sur un catalyseur selon l'invention à base d'oxyde de chrome pauvre ou préalablement appauvri en sels d'ammonium.

Un autre objet de la présente invention consiste en une méthode de préparation d'un catalyseur à base d'oxyde de chrome selon l'invention pauvre en sels d'ammonium, typiquement soit par calcination à une température de 300 à 500°C d'un composé du chrome adéquat, de préférence sous balayage d'un gaz inerte tel que de l'azote, soit par lavage à l'eau de l'oxyde de chrome brut suivi éventuellement d'une étape de mise en oeuvre de l'oxyde de chrome avec d'autres constituants du catalyseur, d'une calcination et d'un traitement au fluorure d'hydrogène.

Un autre objet de la présente invention consiste à fournir un procédé d'hydrofluoration d'hydrocarbures halogénés par action du fluorure d'hydrogène sur un hydrocarbure halogéné en présence d'un tel catalyseur.

Par hydrofluoration, on entend la réaction d'addition de fluorure d'hydrogène sur une double liaison carbone-carbone ainsi que la réaction de substitution d'un atome d'halogène, généralement le chlore ou le brome, par un atome de fluor sur un substrat saturé.

Dans le cadre de la présente invention, les réactions d'hydrofluoration se déroulent sous l'action catalytique du catalyseur à base d'oxyde de chrome introduit comme tel dans le mélange réactionnel ou préalablement fluoré par réaction avec du fluorure d'hydrogène.

L'hydrocarbure halogéné engagé dans le procédé selon l'invention peut être un alcane aliphatique répondant à la formule générale C_{w}HₓX_{y}F_{z} (I) dans laquelle w est un nombre entier compris entre 1 et 6, x est un nombre entier compris entre 0 et (2w+1), y est un nombre entier compris entre 1 et (2w+1), z est un nombre entier compris entre 0 et (2w+1) et la somme (x+y+z) a la valeur (2w+2) et X représente du chlore ou du brome. Avantageusement, l'hydrocarbure halogéné engagé dans le procédé selon l'invention est un alcane aliphatique répondant à la formule (I) dans laquelle w est un nombre entier compris entre 1 et 4, et x est un nombre entier compris entre 1 et 2w.

A titre d'exemples non limitatifs d'alcanes halogénés engagés dans le procédé selon l'invention, on peut citer le dichlorométhane, le chlorofluorométhane, le chlorodifluorométhane, le 1-chloro-1-fluoroéthane, le 1,1-dichloro-1-fluoroéthane, le 1-chloro-1,1-difluoroéthane, les isomères du chlorotétrafluoroéthane, les isomères du dichlorotrifluoroéthane, les isomères du trichlorodifluoroéthane, les isomères du tétrachlorofluoroéthane, le pentachloroéthane, les composés de formule générale C₃H₃Cl_{(5-z})F_{z} et C₄H₅Cl_{(8-z})F_{z} avec z représentant un nombre entier pouvant prendre les valeurs de 1 à 4.

L'hydrocarbure halogéné engagé dans le procédé selon l'invention peut également être un alcène aliphatique répondant à la formule générale C_{w}HₓX_{y}F_{z} (I) dans laquelle w est un nombre entier compris entre 1 et 6, x est un nombre entier compris entre 0 et (2w-1), y est un nombre entier compris entre 1 et (2w-1), z est un nombre entier compris entre 0 et (2w-1), la somme (x+y+z) a la valeur 2w et X représente du chlore ou du brome. L'hydrocarbure halogéné engagé dans le procédé selon l'invention peut également avantageusement être un alcène aliphatique répondant à la formule (I) dans laquelle w est un nombre entier compris entre 1 et 4.

A titre d'exemples non limitatifs d'alcènes halogénés engagés dans le procédé selon l'invention, on peut citer le 1,1-dichloroéthylène, le trichloroéthylène, le perchloroéthylène, le chlorure de vinyle, le 3,3,3-trichloroprop-1-ène, le 1,1,3-trichloroprop-1-ène, le 1,1,3,3-tétrachlorobut-1-ène, le 1,1,1,3-tétrachlorobut-2-ène, le 1,1,1,3-tétrachlorobut-3-ène, le 1,1,4,4,4-pentachlorobut-1-ène, le 1,1,1,3-tétrachloroprop-2-ène, le 1,1,3,3-tétrachloroprop-1-ène, le 1,1,3,3-tétrachloro-2-méthylprop-2-ène, le 1,1,1,3-tétrachloro-2-méthylprop-2-ène, le 1,1,1,3,3-pentachloroprop-2-ène, le 3-chloro-1,1,1-trifluoroprop-2-ène ainsi que les mélanges de ces composés.

L'invention a donc pour but de produire, au départ d'hydrocarbures halogénés saturés ou insaturés, des alcanes fluorés ou chlorofluorés qui contiennent plus d'atomes de fluor et moins d'atomes de chlore que les réactifs engagés. L'invention vise en particulier la synthèse d'hydrocarbures fluorés tels que notamment le difluorométhane, le pentafluoroéthane, le 1,1,1,2-tétrafluoroéthane, le 1,1,1-trifluoroéthane, le 1,1-difluoroéthane, le 2,2-dichloro-1,1,1-trifluoroéthane, le 1,1,1-trifluoro-2-chloroéthane, le 1,1,1,3,3-pentafluoropropane, le 1,1,1,3,3-pentafluorobutane, le 1,1,1,3,3,3-hexafluorobutane, le 1,1,1,3,3-pentafluoro-2-méthylpropane et le 1,1,1,3,3,3-hexafluoropropane. L'invention a plus particulièrement pour but la préparation d'alcanes fluorés ne contenant pas d'atome de chlore sous l'action catalytique d'un oxyde de chrome selon l'invention pauvre en sels d'ammonium.

Plus particulièrement, l'invention a pour but de produire, sous l'action du catalyseur selon l'invention, du pentafluoroéthane par hydrofluoration du perchloroéthylène, du difluorométhane par hydrofluoration du dichlorométhane, du 1,1,1,2-tétrafluoroéthane par hydrofluoration du 2-chloro-1,1,1-trifluoroethane et du 2-chloro-1,1,1-trifluoroéthane par hydrofluoration du trichloroéthylène.

La réaction d'hydrofluoration peut se faire en phase gazeuse ou en phase condensée. La phase gazeuse est préférée.

Généralement, le procédé selon la présente invention est mis en oeuvre de manière continue.

Habituellement, le rapport molaire entre le fluorure d'hydrogène et l'hydrocarbure halogéné mis en oeuvre est supérieur ou égal à 1 et inférieur ou égal à 100. Avantageusement , ce rapport molaire est supérieur ou égal à 3 et inférieur ou égal à 50. D'une manière préférée, ce rapport molaire est supérieur ou égal à 4 et inférieur ou égal à 20.

La pression de réaction n'est pas critique. Habituellement une pression comprise entre 1 et 10 bar convient bien.

Généralement, la température de réaction est comprise entre la température ambiante et 600 °C. Avantageusement, la température de réaction est supérieure ou égale à 100 °C et inférieure ou égale à 500 °C. D'une manière préférée, la température de réaction est supérieure ou égale à 200 °C et inférieure ou égale à 450 °C.

En général, plus la température de réaction est élevée, plus le rapport molaire HF/hydrocarbure halogéné est élevé et plus le temps de contact est long, plus le taux de conversion des réactifs en hydrocarbures fluorés est élevé et plus le taux d'hydrofluoration est important Les paramètres mentionnés ci-dessus peuvent être adaptés de façon à obtenir le produit désiré avec une grande sélectivité ainsi qu'un taux de transformation et un rendement élevés.

Avantageusement, les réactifs non transformés et les composés intermédiaires peuvent être recyclés dans le réacteur d'hydrofluoration pour augmenter la productivité en produit fluoré désiré.

Le procédé selon l'invention peut être mis en oeuvre dans tout type de réacteur ou d'appareillage résistant à la pression, au fluorure d'hydrogène et au chlorure d'hydrogène et, dans le cas d'un procédé en continu, permettant de maintenir en permanence une composition sensiblement stable du mélange réactionnel. Le plus souvent, le procédé selon l'invention est réalisé en continu dans un réacteur phase gazeuse équipé d'un dispositif d'introduction des réactifs, en phase liquide ou gazeuse, et de soutirage d'un courant gazeux, par exemple dans un réacteur tubulaire rempli d'un lit fixe de catalyseur.

Le temps de séjour optimal exprimé comme le rapport entre le débit total des réactifs (à température et pression de réaction) et le volume libre du réacteur peut varier en général de 5 secondes à 10 minutes.

Les exemples ci-après illustrent l'invention de manière non limitative. Dans ces exemples, le taux de transformation de l'hydrocarbure halogéné est le rapport entre la quantité mise en oeuvre diminuée de la quantité non convertie et la quantité mise en oeuvre, multiplié par 100 ; la sélectivité en alcane fluoré ou chlorofluoré est le rapport entre la quantité de l'alcane fluoré ou chlorofluoré formé et la quantité qui aurait été formée si tout l'hydrocarbure halogéné converti avait généré de l'alcane fluoré ou chlorofluoré; la sélectivité globale est la somme de la sélectivité de tous les produits intermédiaires valorisables en alcane fluoré ou chlorofluoré désiré ; le rendement en alcane fluoré ou chlorofluoré est le produit du taux de transformation par la sélectivité en cet alcane fluoré ou chlorofluoré.

### Exemples 1-10

Dans un autoclave cylindrique de 15 mm de diamètre intérieur, on a introduit 20 cm³ d'oxyde de chrome massique renfermant une teneur variable en NH₄⁺ et un mélange fluorure d'hydrogène/-perchloroéthylène (PER) dans un rapport molaire de 10 mol/mol. La pression de réaction a été maintenue à 1 bar et la température à 350 °C. Le temps de séjour était de 12,5 secondes. Le produit principal obtenu est le 1,1,1,2,2-pentafluoroéthane (HFC-125).

Les résultats sont rassemblés dans le tableau I ci-dessous.

**Tableau I**

| N° Essai | [NH₄⁺] (%) | Taux de transformation du PER (%) | Rendement en HFC-125 (% mol) | Sélectivité Globale (% mol) |
|---|---|---|---|---|
| 1(C) | 0,27 | 43 | 12 | 73 |
| 2 | 0,17 | 63 | 30 | 79 |
| 3 | 0,11 | 69 | 34 | 81 |
| 4 | 0,07 | 85 | 47 | 80 |
| 5 | 0,05 | 96 | 58 | 81 |
| 6 | 0,001 | 95 | 58 | 85 |
| 7 | 0,001 | 96 | 59 | 85 |
| 8 | 0,001 | 97 | 60 | 84 |
| 9 (C) | 6.5 | 7 | 0,2 | 85 |

| | | | | |
|---|---|---|---|---|
| (C) indique un exemple comparatif, non conforme à l'invention. | | | | |

## Revendications

1. Catalyseur d'hydrofluoration à base d'oxyde de chrome, contenant des sels d'ammonium la teneur en sels d'ammonium étant inférieure ou égale à 0,2 % en poids, exprimée sous la forme de NH₄⁺ par rapport à la teneur en chrome du catalyseur, exprimée sous la forme de Cr₂O₃.

2. Catalyseur selon la revendication 1 dans lequel la teneur en sels d'ammonium est inférieure ou égale à 0,1 % en poids de sels d'ammonium.

3. Catalyseur selon la revendication 2, dans lequel la teneur en sels d'ammonium est inférieure ou égale à 0,05 % en poids de sels d'ammonium.

4. Catalyseur selon l'une quelconque des revendications 1 à 3, dans lequel l'oxyde de chrome est sous forme massique.

5. Catalyseur selon l'une quelconque des revendications 1 à 4, contenant en outre d'autres métaux ou des sels d'autres métaux et leurs mélanges à titre de co-catalyseur.

6. Procédé d'hydrofluoration d'un hydrocarbure halogéné par réaction avec du fluorure d'hydrogène en présence d'un catalyseur selon l'une quelconque des revendications 1 à 5.

7. Procédé selon la revendication 6 dans lequel l'hydrocarbure halogéné est un alcane aliphatique répondant à la formule générale C_{w}HₓX_{y}F_{z} (I) dans laquelle w est un nombre entier compris entre 1 et 6, x est un nombre entier compris entre 0 et (2w+1), y est un nombre entier compris entre 1 et (2w+1), z est un nombre entier compris entre 0 et (2w+1), la somme (x+y+z) a la valeur (2w+2) et X représente du chlore ou du brome.

8. Procédé selon la revendication 6 dans lequel l'hydrocarbure halogéné est un alcène aliphatique répondant à la formule générale C_{w}HₓX_{y}F_{z} (I) dans laquelle w est un nombre entier compris entre 1 et 6, x est un nombre entier compris entre 0 et (2w-1), y est un nombre entier compris entre 1 et (2w-1), z est un nombre entier compris entre 0 et (2w-1), la somme (x+y+z) a la valeur 2w et X représente du chlore ou du brome.

9. Procédé selon l'une quelconque des revendications 6 à 8 dans lequel la réaction de l'hydrocarbure halogéné avec le fluorure d'hydrogène se déroule en phase gazeuse.

10. Procédé selon l'une quelconque des revendications 6 à 9 pour la synthèse du pentafluoroéthane par réaction entre le fluorure d'hydrogène et un composé choisi parmi le perchloroéthylène, le fluorotétrachloroéthane, le difluorotrichloroéthane, le trifluorodichloroéthane et le chlorotétrafluoroéthane.

11. Procédé selon l'une quelconque des revendications 6 à 9 pour la synthèse du difluorométhane par réaction entre le fluorure d'hydrogène et le dichlorométhane.

12. Procédé selon l'une quelconque des revendications 6 à 9 pour la synthèse du 1,1,1,2-tétrafluoroéthane par réaction entre le fluorure d'hydrogène et un composé choisi parmi le trichloroéthylène et le 2-chloro-1,1,1-trifluoroéthane.

## Patentansprüche

1. Hydrofluorierungskatalysator auf Chromoxidbasis mit einem Gehalt an Ammoniumsalzen, worin der Gehalt an Ammoniumsalzen kleiner oder gleich 0,2 Gew.-%, ausgedrückt in Form von NH₄⁺, bezogen auf den Chromgehalt des Katalysators, ausgedrückt als Cr₂O₃, ist.

2. Katalysator nach Anspruch 1, worin der Gehalt an Ammoniumsalzen kleiner oder gleich 0,1 Gew.-% an Ammoniumsalzen ist.

3. Katalysator nach Anspruch 2, worin der Gehalt an Ammoniumsalzen kleiner oder gleich 0,05 Gew.-% an Ammoniumsalzen ist.

4. Katalysator nach einem der Ansprüche 1 bis 3, worin das Chromoxid in Masseform vorliegt.

5. Katalysator nach einem der Ansprüche 1 bis 4, der zusätzlich andere Metalle oder Salze anderer Metalle und deren Gemische als Co-Katalysator enthält.

6. Verfahren zur Hydrofluorierung eines halogenierten Kohlenwasserstoffes durch Umsetzung mit Fluorwasserstoff in Gegenwart eines Katalysators nach einem der Ansprüche 1 bis 5.

7. Verfahren nach Anspruch 6, worin der halogenierte Kohlenwasserstoff ein aliphatisches Alkan entsprechend der allgemeinen Formel C_{w}HₓX_{y}F_{z} (I) ist, worin w eine ganze Zahl von 1 bis 6 darstellt, x eine ganze Zahl von 0 bis (2w+1) ist, y eine ganze Zahl von 1 bis (2w+1) ist, z eine ganze Zahl von 0 bis (2w+1) ist, die Summe (x+y+z) den Wert (2w+2) hat und X für Chlor oder Brom steht.

8. Verfahren nach Anspruch 6, worin der halogenierte Kohlenwasserstoff ein aliphatisches Alken entsprechend der allgemeinen Formel C_{w}HₓX_{y}F_{z} (I) ist, worin w eine ganze Zahl von 1 bis 6 darstellt, x eine ganze Zahl von 0 bis (2w-1) ist, y eine ganze Zahl von 1 bis (2w-1) ist, z eine ganze Zahl von 0 bis (2w-1) ist, die Summe (x+y+z) den Wert 2w hat und X für Chlor oder Brom steht.

9. Verfahren nach einem der Ansprüche 6 bis 8, worin die Umsetzung des halogenierten Kohlenwasserstoffes mit Fluorwasserstoff in gasförmige Phase erfolgt.

10. Verfahren nach einem der Ansprüche 6 bis 9 zur Herstellung von Pentafluorethan durch Umsetzung von Fluorwasserstoff mit einer unter Perchlorethylen, Fluortetrachlorethan, Difluortrichlorethan, Trifluordichlorethan und Chlortetrafluorethan ausgewählten Verbindung.

11. Verfahren nach einem der Ansprüche 6 bis 9 zur Herstellung von Difluormethan durch Umsetzung von Fluorwasserstoff mit Dichlormethan.

12. Verfahren nach einem der Ansprüche 6 bis 9 zur Herstellung von 1,1,1,2-Tetrafluorethan durch Umsetzung von Fluorwasserstoff mit einer unter Trichlorethylen und 2-Chlor-1,1,1-trifluorethan ausgewählten Verbindung.

## Claims

1. Hydrofluorination catalyst based on chromium oxide containing ammonium salts and which exhibits a content of ammonium salts of less than or equal to 0.2% by weight, expressed in the form of NH₄⁺, with respect to the content of chromium in the catalyst, expressed in the form of Cr₂O₃.

2. Catalyst according to Claim 1, in which the content of ammonium salts is less than or equal to 0.1% by weight of ammonium salts.

3. Catalyst according to Claim 2, in which the content of ammonium salts is less than or equal to 0.05% by weight of ammonium salts.

4. Catalyst according to anyone of Claims 1 to 3, wherein the chromium oxide is in bulk form.

5. Catalyst according to anyone of Claims 1 to 4, additionally comprising other metals or salts of other metals and their mixtures as cocatalyst.

6. Process for the hydrofluorination of a halogenated hydrocarbon by reaction with hydrogen fluoride in the presence of a catalyst according to either one of Claims 1 to 5.

7. Process according to Claim 6, in which the halogenated hydrocarbon is an aliphatic alkane corresponding to the general formula C_{w}HₓX_{y}F_{z} (I), in which w is an integer between 1 and 6, x is an integer between 0 and (2w + 1), y is an integer between 1 and (2w + 1), z is an integer between 0 and (2w + 1), the sum (x + y + z) has the value (2w + 2) and X represents chlorine or bromine.

8. Process according to Claim 6, in which the halogenated hydrocarbon is an aliphatic alkene corresponding to the general formula C_{w}HₓX_{y}F_{z} (I), in which w is an integer between 1 and 6, x is an integer between 0 and (2w - 1), y is an integer between 1 and (2w - 1), z is an integer between 0 and (2w - 1), the sum (x + y + z) has the value 2w and X represents chlorine or bromine.

9. Process according to any one of Claims 6 to 8, in which the reaction of the halogenated hydrocarbon with the hydrogen fluoride takes place in the gas phase.

10. Process according to any one of Claims 6 to 9 for the synthesis of pentafluoroethane by reaction between hydrogen fluoride and a compound chosen from perchloroethylene, fluorotetrachlorethane, difluorotrichloroethane, trifluorodichloroethane and chlorotetrafluoroethane.

11. Process according to any one of Claims 6 to 9 for the synthesis of difluoromethane by reaction between hydrogen fluoride and dichloromethane.

12. Process according to any one of Claims 6 to 9 for the synthesis of 1,1,1,2-tetrafluoroethane by reaction between hydrogen fluoride and a compound chosen from trichloroethylene and 2-chloro-1,1,1-trifluoroethane.
